# FASCICULE DE BREVET EUROPEEN

(11) **EP 0 573 536 B1**
(45) Date de publication et mention de la délivrance du brevet: **23.04.1997**
(21) Numéro de dépôt: 92906385.7
(22) Date de dépôt: 28.01.1992
(51) Int. Cl.: C12N 1/20, C12N 9/24, D21C 9/10

(54) **XYLANASE, SOUCHES DE BACILLUS PRODUCTRICES DE XYLANASE ET LEURS UTILISATIONS**
XYLANASE, BAZILLUSSTÄMME DIE XYLANASE PRODUZIEREN UND IHRE VERWENDUNG
XYLANASE, XYLANASE-PRODUCING BACILLUS AND APPLICATIONS THEREOF

(30) Priorité: 01.02.1991 FR 9101191
(43) Date de publication de la demande: 15.12.1993
(73) Titulaire: INSTITUT NATIONAL DE LA RECHERCHE AGRONOMIQUE (INRA), F-75338 Paris Cédex 07 (FR)
(72) Inventeur: SAMAIN, Eric, F-38610 Gières (FR); DEBEIRE, Philippe, F-59200 Tourcoing (FR); DEBEIRE-GOSSELIN, Michèle, F-59200 Tourcoing (FR); TOUZEL, Jean-Pierre, F-59650 Villeneuve-d'Ascq (FR)
(74) Mandataire: Phélip, Bruno
(86) Numéro de dépôt international: FR9200077
(87) Numéro de publication internationale: WO9213942

(56) Documents cités:
- PATENT ABSTRACTS OF JAPAN, vol. 014, no. 106 (C-694), 27 Février 1990
- APPLIED MICROBIOLOGY AND BIOTECHNOLOGY, vol. 34, no. 1, Octobre 1990, BERLIN, DE, pages 141 - 144; S.RAJARAM ET AL.: 'Production and characterization of xylanase from Bacillus thermoalkalophilus grown on agricultural wastes'
- WORLD PATENTS INDEX LATEST, Week 2085, Derwent Publications Ltd., London, GB; AN 85-118644
- ENZYME MICROBIOLOGY AND TECHNOLOGY, vol. 8, Mai 1986, HAYWARDS HEATH, GB, pages 309-314; H.GRÜNINGER ET AL.: 'A novel,highly thermostable D-xylanase'
- AGRICULTURAL AND BIOLOGICAL CHEMISTRY, vol. 45, Mai 1981, TOKYO, JP, pages 1121-1127; F.UCHINO ET AL.: 'A thermostable xylanase from a thermophillic acidophillic Bacillus sp.'
- ABSTRACT BULLETIN OF THE INSTITUTE OF PAPER CHEMISTRY, vol. 59, no. 8, Février 1989, APPLETON, US, page 864; J-M.CHAUVET ET AL.: 'Assistance in bleaching of never-dried pulps by the use of xylanases,consequences on pulp properties'

## Description

La présente invention a pour objet une xylanase, ainsi que des souches de Bacillus productrices de cette enzyme.

Elle concerne également l'utilisation de cette enzyme et de ces bactéries dans le blanchiment de la pâte à papier et la préparation de xylose ou de xylo-oligosaccharides à partir de matières premières végétales , notamment .

Des utilisations variées des xylanases ont été proposées dans le domaine des biotechnologies, en particulier dans le domaine alimentaire ( Biely, Trends Biotechnol 3 (11) : 286-290, 1985 ) , dans l'industrie du papier ( Mora et al., J. Wood Chem.Technol, 6: 147-165, 1986) ou dans la production de composés chimiques à partir de l'hémicellulose (Reilly P.J., 1981, Xylanases : structure and function in Trends in the Biology of Fermentations for Fuels and Chemicals. A.J. Hollaender (Ed). Plenum, New York).

La faisabilité technique de telles applications a été principalement évaluée à l'aide d'enzymes produites par des champignons mésophiles. Cependant , de telles applications pourraient être facilitées par l'utilisation de champignons présentant une meilleure stabilité à la température .

Diverses bactéries et enzymes sont connues pour la production de xylanases ( voir notamment Wong et al., Microbiological Reviews , 52, n°3, 305-317, 1988). Jusqu'à maintenant, les plus hauts rendements en enzymes ont été obtenus à partir de champignons (Yu et al. Enzyme Microb. Technol. 9:16-24, 1987). Cependant, des souches hyperproductrices de Bacillus, ont été déjà décrites (Okazaki et al. Appl. Microbiol.Biotechnology , 19: 335-340, 1984; Okazaki et al., Agric. Biol. Chem. 49, 2033-2039, 1985) . De telles espèces de Bacillus thermophiles et dégradant le xylane peuvent être de bons candidats pour la production industrielle de xylanases, en raison de leur taux de croissance important et d'une bonne connaissance de leur génétique.

Les xylanases isolées par Okazaki et al. sont issues de deux souches de Bacillus , appelées W1 et W2 par les auteurs . Dans chacune de ces souches , on a mis en évidence deux composants de l'activité xylanasique appelées I et II. Les composants I dégradent le xylane en xylobiose et en oligomères de degré de polymérisation supérieur, tandis que les composants II produisent en plus des composés précédents, de xylose.

Les composants I (appelés W1 I et W2.I) ont des poids moléculaires respectifs de 21,5 kDa et 22,5 kDa ainsi que des points isoélectriques de 8,5 et 8,3. Les composants II (W1.II et W2.II) ont quant à eux des poids moléculaires respectifs de 49,5 kDa et 50 kDa .

Les deux composants I et II sont inhibés par les ions Hg²⁺, et à un moindre degré par Cu^{2+.}

De nombreuses autres xylanases ont été isolées à partir de diverses espèces de Bacillus , de Clostridium , d'Aspergillus, de Streptomyces ou de Trichoderma entre autres ( Wong et al., 1988 précédemment cités ).

Ainsi , le résumé du brevet japonais JP 130 96 84 ( RIKAGAKU KENKYSHO) concerne une xylanase de type WII ayant un poids moléculaire de 50 kD ou 42 kD. Aucun point isoélectrique n'est mentionné pour cette xylanase .

Un article de RAJARAM et al. ( Applied Microbiology and Biotechnology , vol.34 , n°1 , Octobre 1990, pages 141-144) est relatif à une souche de Bacillus isolée dans la nature et produisant une xylanase ayant une activité optimale entre 60°C et 70°C et à un pH compris entre 6 et 7 . Cette enzyme n'est caractérisée ni par son poids moléculaire ni par son point isoélectrique . Cette souche produit de plus d'autres enzymes telles que des cellulases.

Un autre résumé de brevet japonais au nom de RIKAGAKU KENKYUSHO ( JP-85 118 644 ) décrit une xylanaseayant une activité optimale à un pH compris entre 6 et 7 . Cet enzyme aurait un poids moléculaire déterminé par ultrafiltration compris entre 50 et 100 kD. Aucun point isoélectrique n'est mentionné dans ce résumé .

Un article de GRÜNINGER et al. ( Enzyme Microbiology and Technology , Vol.8, mai 1986 , pages 309-314 ) concerne une autre souche de Bacillus isolée à partir de boue et produisant une xylanase thermostable . L'enzyme est caractérisée comme ayant une activité optimale à 78°C et à un pH d'une valeur de 7,5 . Cette enzyme n'est caractérisée ni par son poids moléculaire ni par son pH isoélectrique .

La production industrielle de xylanases se heurte à la présence simultanée d'activités contaminantes telles que les cellulases, entraînant des surcoûts de purification. D'autre part, l'utilisation de germes génétiquement modifiés peut présenter des problèmes tels que l'instabilité des plasmides portant les gènes codant pour les xylanases.

A la connaissance du demandeur , la meilleure productivité en endoxylanase obtenue par un microorganisme ne produisant pas de cellulase l'a été à partir d'un mutant de Streptomyces lividans dépourvu d'activité cellulosique après introduction d'un plasmide porteur des gènes codant pour les xylanases A et B. Des productivités de l'ordre de 6000 à 10.000 U.I. l/h ont été observées dans les milieux de culture. Il faut néanmoins noter que dans ce cas des problèmes liés à la non-hydrolyse du xylane insoluble par la xylanase A et de stabilité thermique pour la xylanase B ont été rencontrés . ( Kluepfel et al. Biochem. J. 267, 47-50, 1990).

Aucune des enzymes décrites dans l'art antérieur ne présentait donc , à la connaissance du demandeur , des caractères permettant une application industrielle , c'est-à-dire une bonne stabilité thermique, une aptitude importante à la dégradation des substrats et une obtention par des souches superproductrices .

Le demandeur a donc montré de manière surprenante que certaines souches de Bacillus produisaient des xylanases thermostables et présentant une activité de dégradation importante.

De manière encore plus surprenante, le demandeur a montré que ces souches de Bacillus excrétaient en grande quantité de telles enzymes , sans produire de quantités notables de cellulases .

La présente invention a donc pour objet des souches de Bacillus hyper productrices en xylanase, et en particulier les souches enregistrées sous les n° I-1017 et I-1018 auprès de la Collection Nationale de Cultures des Microorganismes de l'Institut Pasteur.

Ces souches ont été isolées à partir de fumier.

Les bactéries se présentent comme des bâtonnets courts et droits formant des spores et ayant une taille moyenne de 0,5 µ à 2,5 µ . Elles sont généralement seules ou appariées, présentent une coloration gram positive et les spores sont ovales et centrales.

Ces souches sont strictement aérobies, aucune croissance n'étant observée en l'absence d'oxygène et le nitrate n'est pas utilisé en tant qu'accepteur d'électrons.

Le pH optimal de croissance et de production de xylanase est de 7,8 tandis que les pH limites pour la croissance se situent entre 6,5 et 8,5 et la température maximale est de 63°C.

Outre le xylane, ces souches utilisent le xylose, le glucose, le saccharose, le maltose ainsi que l'amidon. Le fructose , l'arabinose, la cellulose, la pectine ainsi que la chitine ne sont pas utilisées.De plus , la croissance est inhibée par une concentration de 5% en chlorure de sodium.

Le contenu en G+C de l'ADN de ces souches déterminé par dénaturation thermique est de 57,5% en mole .

Ces souches sont bien différenciées des autres souches productrices de xylanases, comme indiqué sur le tableau I , du fait des temps de culture et de leur productivité en xylanase différents.

La souche I-1018 a été obtenue par mutagénèse de la souche I-1017. La souche I-1018 possède les mêmes caractéristiques générales que la souche I-1017, mais présente une hyperproduction en xylanase.

La présente invention a d'autre part pour objet une xylanase thermophile (stable à 60°C environ) caracterisée en ce qu'elle possède une masse moléculaire de 22 kDa environ, et un point isoélectrique d'environ 7,7 et en ce qu'elle présente la séquence d'acides aminés N-terminale suivante:

Asn-Thr-Tyr-Trp-Gln-Tyr-Trp-Thr-Asp-Gly-Ile-Gly-Tyr-
Val-Asn-Ala-Thr-Asn-Gly-Gln.

Cette enzyme présente d'autre part avantageusement une grande stabilité à 60°C, pendant au moins 24 heures, et un pH d'activité optimale compris dans la gamme allant de 4,8 à 7, et préférentiellement d'environ 6.

Il est à noter que le pHi de cette enzyme est assez élevé mais néanmoins inférieur au pHi des xylanases de masse moléculaire voisine produites par des Bacillus, notamment celles décrites par Okazaki et al. (1985, publication précédemment citée).

Le pH 6 correspond à un pH optimal, mais l'activité reste supérieure à 80% dans la gamme comprise entre 4,8 et 7.

Avantageusement, elle comprend dans sa séquence 15 alanines, 6 arginines, 29 aspartates et asparagines, 19 glutamates et glutamines, 31 glycines, 3 histidines, 6 isoleucines, 7 leucines, 3 lysines, 1 méthionine, 4 phénylalanines, 8 prolines, 19 sérines, 13 thréonines, 22 tyrosines et 14 valines.

Cette enzyme est de plus inhibée par Hg²⁺' mais ne l'est pas par Ag²⁺ ( 1 mM), ni par le SDS à 0,1%.

La constante de Michaelis (Km) vis-à-vis du xylane de bouleau est de 0,9 g/l.

Les oligosaccharides neutres produits par cette enzyme à partir de xylane de bouleau ( 5 g/l de xylane de bouleau ; 500 U/l , 60°C) sont composés :
- pour des temps très courts d'incubation enzymatique ( 15 minutes ) , de xylotriose et de xylo-oligosaccharides de degrés de polymérisation supérieurs,
- pour des temps d'incubation longs ( 24 à 72 heures) de xylobiose, de xylotriose et de xylotétraose, ce dernier en très faible quantité.

Cette endoxylanase est notamment excrétée par le Bacillus, et en particulier par les souches I-1017 et I-1018 de Bacillus précédemment décrites.

Elle est excrétée dans le milieu de culture.

Le milieu de culture de ces souches est notamment caractérisé par une absence d'activité endo-β-1,4-glucanasique ( cellulase) et par la présence d'une très faible activité β-xylosidasique contaminante (environ 0,06 U.I./ml de surnageant de culture pour les deux souches ) . Il est à noter que le surnageant de culture peut être lyophilisé sans perte notable d'activité xylanasique et se conserve congelé pendant au moins un an sans perte d'activité.

Les activités endo-β-1,4-glucanasiques ont été déterminées par la mesure des sucres réducteurs libérés à partir de carboxyméthylcellulose (CMC) à 1% ou d'Avicel (1%) dans du tampon acétate de sodium 50mM pH 6. L'activité β-xylosidasique a été déterminée par la mesure de la libération des p-nitrophénol à partir de p-nitrophényl-β-D-xyloside (0.1%) dans du tampon acétate de sodium 50 mM pH 6.

Une unité d'activité xylanasique (U.I.) est définie comme une micromole d'équivalent xylose réducteur libéré par minute à 60°C.

La concentration de sucre réducteur est déterminée par la méthode de Kidby et Davidson (Anal. Biochem., 55:321-325, 1973).

L'enzyme purifiée quant à elle se conserve sous forme congelée dans l'éthylèneglycol à 20% sans perte notable d'activité pendant un an.

La présente invention a encore pour objet un procédé d'obtention de la xylanase telle que précédemment décrite comprenant les étapes suivantes :
- concentration du surnageant de culture de Bacillus n° I-1017 ou I-1010 par ultrafiltration,
- passage sur colonne échangeuse d'ions, telle qu'une colonne de O-Sépharose Fast Flow, (Pharmacia),
- passage sur colonne d'interactions hydrophobes,telle qu'une colonne de phényle sépharose (Pharmacia).

La concentration du surnageant peut notamment être effectuée par ultrafiltration sur membrane de polysulfone à seuil d'exclusion supérieur à 10 kDa.

Ce procédé permet d'obtenir une préparation de xylanase substantiellement pure.

La présente demande est d'autre part relative à un procédé de production de la xylanase précédemment décrite comprenant les étapes :
- de croissance des bactéries n° I-1017 ou I-1010 dans un milieu contenant un substrat de croissance tel que du glucose, et
- de production de xylanase induite par alimentation en continu de la culture par des xylo-oligosaccharides en quantités appropriées.

La présente invention a également pour objet i'utilisation de la xylanase précédemment décrite dans le blanchiment de la pâte à papier.

Un avantage de cette xylanase réside dans le fait que le degré d'hydratation de la pâte à papier a peu d'importance. Il n'est pas obligatoire de diluer beaucoup la pâte pour obtenir une bonne attaque enzymatique. L'utilisation de cette xylanase comme auxiliaire dans le blanchiment de la pâte à papier est d'autant plus intéressante que les préparations sont dépourvues de contaminants cellulasiques.

Cette xylanase peut aussi être utilisée pour la préparation de xylose ou de xylo-oligosaccharides à partir de matières premières d'origine végétale, qui sont des matières premières peu coûteuses et renouvelables ( par exemple rafles de maïs).

D'autres utilisations des xylanases ont été mentionnées dans la littérature . La revue de Zeikus et al. (Thermostable saccharidases New Sources uses and Biodesigns in " Enzymes in biomass conversion ", Leatham and Himmel , ACS Washington D.C. , 1991) répertorie les principales utilisations des xylanases. Elles sont principalement utilisées dans la fabrication d'aliments où leurs propriétés permettent d'améliorer la panification , la clarification de jus de fruits et de vins et les qualités nutritionnelles des fibres des céréales et dans la production d'épaississants pour l'alimentation.

La deuxième gamme d'application concerne les industries de la pulpe de papier et des fibres, où elles utilisées pour le blanchissement des pâtes, la fabrication de pâte de bois et la purification de fibres pour la fabrication de rayonne .

On note aussi des utilisations dans l'alimentation pour les volailles dans lesquelles les xylanases sont utilisées afin de diminuer la viscosité des aliments ( Van Paridon et al. Xylans and Xylanases, International Symposium, Wageningen, 8-11 Décembre 1991 ; Bedford et Classen H.L. Xylans and Xylanases, International Symposium, Wageningen, 8-11 Décembre 1991 ).

L'utilisation de xylanases dans la valorisation de sous-produits de l'industrie de la pâte à papier est plus précisément mentionnée dans l'article de Biely ( Trends in Biotechnology , Vol.3, n° 11, 1985 ).

On peut aussi citer les deux brevets européens EP-228.732 et EP-227.159 qui concernent respectivement l'utilisation de xylanases pour améliorer la filtrabilité de sirop de glucose et de bière .

On notera aussi la possibilité d'utiliser des xylanases pour la production de composés chimiques à partir de l'hémicellulose ( Reilly , précédemment cité) .

Ces diverses publications montrent que les xylanases objets de la présente invention peuvent être utilisées dans de nombreuses applications .

La présente invention est illustrée sans être pour autant limitée par les exemples d'applications qui suivent .

La figure 1 représente l'évolution de cultures discontinues de la souche I-1017.

La figure 2 représente l'évolution d'une culture en continu de la souche I-1017 alimentée en continu par des solutions de xylo-oligosaccharides à différentes concentrations.

La figure 3 représente la production de xylanases comparée des souches I-1017 et I-1018 en présence de différentes concentrations de xylo-oligosaccharides.

La figure 4 représente la libération de xylo-oligosaccharides, à partir de pâte Kraft non blanchie , par différentes concentrations de xylanase.

La figure 5 représente la libération de xylo-oligosaccharides à partir de rafles de maïs broyées.

### EXEMPLE 1-

### Sélection de la souche I-1017:

Différents échantillons de sol, de compost et de fumier ont été utilisés comme inoculum afin d'enrichir en souches xylanolytiques . Après 24 heures d'incubation, les activités xylanasiques sont testées dans les surnageants de culture, et les trois enrichissements montrant les activités les plus importantes sont dilués et étalés sur du milieu agar contenant du xylane.

Un milieu de culture de base contenant des minéraux et des vitamines (Zeikus et Wolfe, J. Bacteriol.109: 707-713, 1972) et de l'extrait de levure (0,2%) a été complémenté avec de l'agar (2%) et du xylane d'avoine (0,5%) . Après stérilisation, du KHCO₃ (25 mM) est ajouté à partir d'une solution stock stérile 1M au milieu d'isolement des souches mélangé . Les boîtes de Pétri ensemencées sont incubées à 55°C dans des jarres fermées.

Les colonies formant des zones claires sont purifiées . Les isolats les plus producteurs (souche I-1017) sont sélectionnés et caractérisés.

L'enrichissement ainsi que la croissance des cultures sont effectués de manière aérobie dans un bain-marie agité dans des bouteilles fermées de 125 ml contenant 10 ml de milieu. La température d'incubation est de 55°C et le milieu est tamponné à pH 7 par addition de KHCO₃ 50 mM.

### EXEMPLE 2:

### Croissance de la souche I-1017.

La cinétique de production des xylanases est effectuée à 55°C dans des fermenteurs de 2 litres dans un volume initial de milieu de 1,5 litre . Après stérilisation, 50 ml d'une solution 1M KHCO₃ sont ajoutés et le pH est ajusté à 7,8 et maintenu à cette valeur . La concentration d'oxygène dissous est régulée et maintenue à un taux de saturation de 70%.

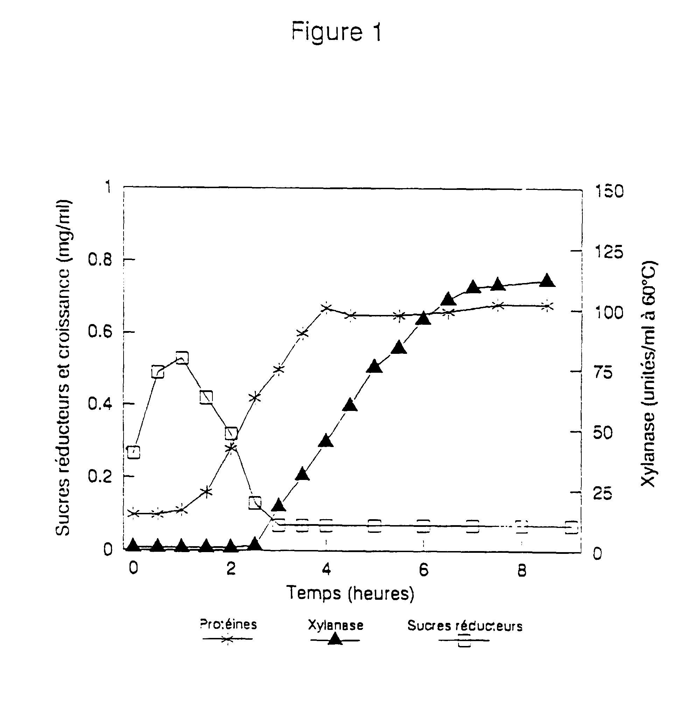

La figure 1 représente la croissance de la souche I-1017 en culture discontinue sur du xylane d'avoine à 0,5 % . La souche I-1017 produit jusqu'à 110 unités de xylanase/ml. Durant les premières heures de culture, une accumulation transitoire de sucres réducteurs est observée . Cette accumulation est due à la présence dans l'inoculum de petites quantités de xylanase qui catalysent une hydrolyse rapide de la fraction soluble de xylane en xylo-oligosaccharides.

La croissance bactérienne permet alors la consommation des xylo-oligosaccharides et la synthèse de xylanase qui débute juste après que leur concentration devienne limitante pour la croissance des bactéries.

La production de xylanase est à peu près constante durant 4 heures ( 30 U.I. ml/h).

On observe une grande variabilité dans la production de xylanase en fonction de l'activité xylanase préalable de l'inoculum, de l'importance de la phase stationnaire et des caractéristiques physiques du xylane.

b) Influence de la quantité de xylo-oligosaccharides sur la croissance de la souche I-1017.

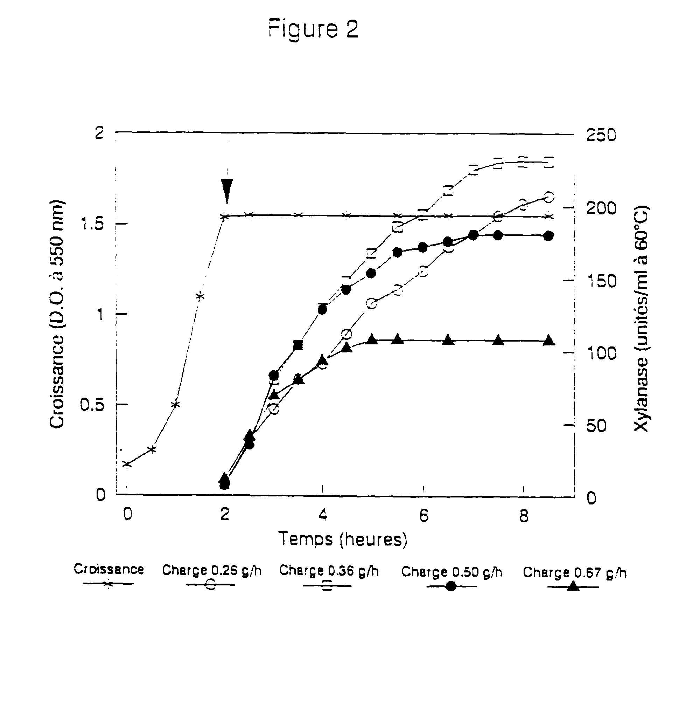

Afin d'obtenir des conditions de culture reproductibles , la production de xylanase a été testée dans des cultures alimentées par une solution en présence de xylo-oligosaccharides . La figure 2 montre l'influence de la concentration en xylo-oligosaccharides.

La concentration en sucre initiale est de 2 g/l . Le substrat est consommé pendant la phase de croissance exponentielle durant laquelle aucune production de xylanase n'est détectée.

Quand la concentration de substrat commence à devenir limitante pour la croissance , on observe une synthèse de xylanase immédiate.

Le taux et aussi la durée de production de l'enzyme sont dépendants de la fourniture de substrat qui varie de 0,26 g/h à 0,67 g/h , avec un flux de substrat de 24 ml/h et un volume initial du milieu de culture de 1,5 1.

La flèche sur la figure 2 correspond à l'addition des xylo-oligosaccharides.

La production d'enzymes la plus importante (230 U.I./ml) est obtenue quand on fournit le substrat à un taux de 0,36 g/h . Des quantités de substrats supérieures entraînent un raccourcissement de la période de production des xylanases.

Quand le glucose est utilisé à la place de xylo-oligosaccharides, la production de xylanase est considérablement diminuée ( 14 U.I./ml) et dure moins de 2 heures.

D'autre part, l'utilisation de glucose comme substrat initial de croissance ne modifie pas le rendement en xylanase par comparaison à l'utilisation de xylo-oligosaccharides .

Afin de préparer les xylo-oligosaccharides comme substrats dans des cultures en continu, on incube une suspension de xylane d'avoine à 5% durant 8 heures à 60°C et à pH6 en présence de 5 unités de xylanase par ml. Cette suspension est ensuite centrifugée et le surnageant est récupéré puis autoclavé.

### EXEMPLE 3:

### Purification de la xylanase à partir de culture de la souche I-1017.

Le procédé de purification de la xylanase comprend les étapes suivantes :
- concentration du surnageant de culture de Bacillus, par ultrafiltration sur membrane de polysulfone à seuil d'exclusion de 10 kDa,
- passage sur colonne échangeuse d'ions Sépharose Fast Flow , ( Pharmacia ),
- passage sur colonne d'interactions hydrophobes phényle Sépharose (Pharmacia).

Les résultats de cette purification sont rassemblés dans le tableau II.

Cette purification montre que l'endoxylanase est la protéine majeure parmi les protéines excrétées dans le milieu de culture (environ 50% des protéines totales excrétées ).

### EXEMPLE 4 :

### Caractérisation de la xylanase.

40 µg de xylanase purifiée selon l'exemple 3 ont été séquencés à l'aide d'un séquenceur en phase gazeuse de type Applied Biosystems 470 A. Les dérivés phénylthiohydantoïne (PTH) de chaque acide aminé ont été identifiés par chromatographie liquide à haute pression (CLHP) à l'aide d'un analyseur de PTH couplé à un appareil Applied Biosystems 120 A.

La séquence N-terminale des 20 premiers acides aminés obtenue est la suivante :

Asn-Thr-Tyr-Trp-Gln-Tyr-Trp-Thr-Asp-Gly-Ile-Gly-Tyr-
Val-Asn-Ala-Thr-Asn-Gly-Gln.

La composition globale en acides aminés a aussi été déterminée après hydrolyse à l'acide chlorhydrique 5,6 N à 100°C durant 24 heures.

Les résultats sont indiqués dans le tableau III,dans lequel on a fait figurer le pourcentage molaire de chaque acide aminé, le nombre estimé de résidus de chaque type dans la protéine et par comparaison le nombre de résidus de chaque type dans l'extrémité N-terminale de 20 acides aminés.

On remarque que les résidus asparagine et glutamine ont été comptés respectivement avec les résidus aspartate et glutamate , et que les résidus tryptophane et cystéine n'ont pas été mesurés.

### EXEMPLE 5:

### Mutagénèse de la souche I-1017.

Des cultures de la souche I-1017 ont été effectuées sur un milieu contenant du xylane et de l'éthylméthane sulfonate (EMS).

Les cellules traitées sont lavées deux fois, incubées toute la nuit et étalées sur un milieu agarxylane .

Les clones sont cultivés dans des microtubes en verre ( 8 x 35 mm ) remplis avec 100 microlitres d'un milieu de culture complémenté avec du xylane d'avoine ( 0,5 %) . Les microtubes sont fermés à l'aide de bouchons de silicone afin d'éviter l'évaporation et incubés durant 24 heures à 55°C sur agitateur.

Pour la sélection de mutants 96 échantillons sont testés en parallèle; 20 µl de chaque culture de microtube sont transférés à l'aide d'une pipette multicanaux dans des microtubes de polypropylène où l'hydrolyse du xylane est effectuée à la température ambiante. Les réactions sont initiées par l'addition de 300 µl de suspension de substrat dans chaque tube et arrêtées par 400 µl d'acide 3,5-dinitrosalicylique.Après ébullition durant 15 minutes, 50 µl de chaque tube sont transférés dans une plaque de microtitration afin de lire la densité optique.

Les clones montrant les activités xylanasiques les plus importantes sont retestés par cinq repiquages successifs et comparés avec la souche parentale.

Des mutants ayant un accroissement significatif d'activité xylanase ont été sélectionnés parmi 500 clones isolés après mutagénèse avec l'EMS.

Ces mutants montrent une augmentation de l'activité xylanase dans des cultures discontinues sur milieu contenant du xylane.

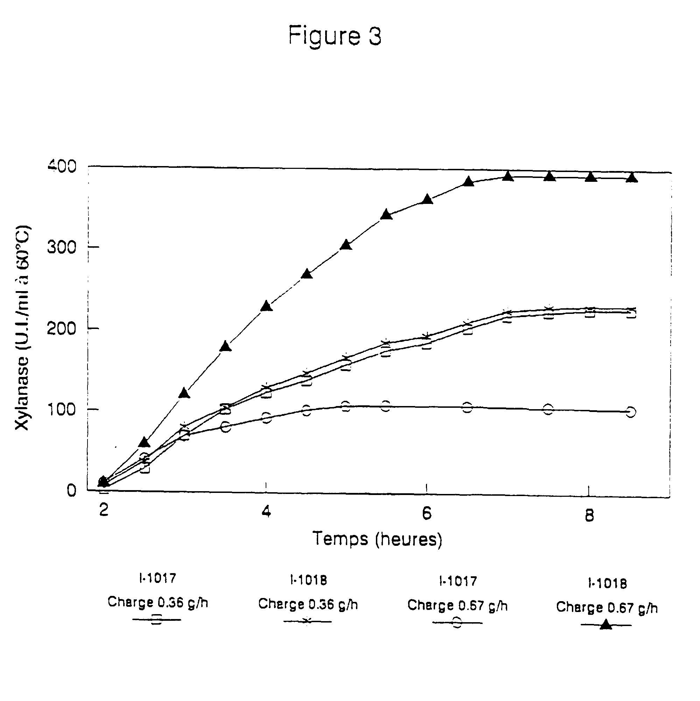

La productivité de xylanase des mutants est aussi déterminée dans des cultures alimentées en continu. Avec un flux de xylo-oligosaccharides de 0,36 g/h , le rendement de xylanase du mutant I-1018 n'est pas augmenté de manière significative ( moins de 10%) par rapport à celui de la souche sauvage I-1017 comme le montre la figure 3 qui montre la croissance différentielle des souches I-1017 et I-1018 à deux concentrations en xylo-oligosaccharides.

Quand le flux en xylo-oligosaccharides est augmenté à 0,67 g/l, la souche I-1018 par exemple montre une augmentation de deux fois supérieure de la production de xylanase par rapport à la souche I-1017 (souche parentale ).

L'obtention de mutant et l'optimisation des conditions de culture alimentées en continues permettent une augmentation d'un facteur 3 dans la production de xylanase par comparaison avec les cultures en discontinu de la souche sauvage sur du xylane . Ces taux d'enzymes élevés ( jusqu'à 392 U.I./ml) sont obtenus en 7 heures de culture et la productivité de xylanase, par I-1018 est donc considérablement supérieure à celle des meilleures souches de champignons et de bactéries ( Bertrand et al., Biotechnol. Bioeng., 33:791-794, 1989 ) (Yu et al. 1987 précédemment cité)productrices de xylanase qui nécessitent plusieurs jours avant d'obtenir de tels taux d'enzymes.

### EXEMPLE 6:

### Traitement de pâtes à papier ( Kraft non blanchies) par la xylanase.

On a traité 8.5 g de pâte humide (correspondant à 1 g de poids sec ) provenant de pin maritime et renfermant 60 mg de xylane , par 25 ml de tampon (contenant diverses concentrations de xylanase) à 60°C pendant 24 heures.

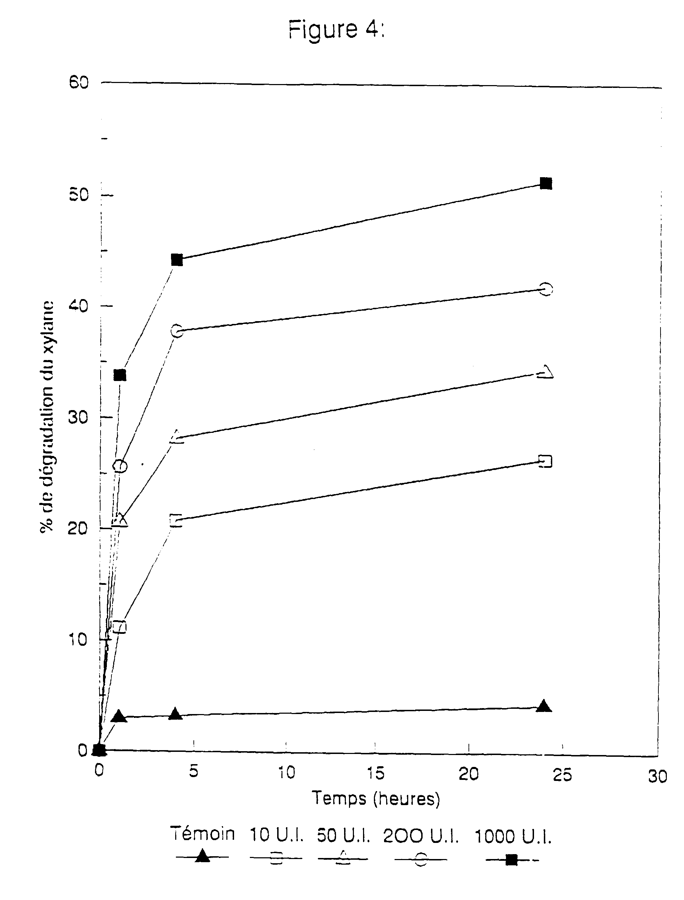

Les cinétiques de libération de xylo-oligosaccharides sont représentées à la figure 4. On a montré une libération conjointe de dérivés de la lignine ( par mesure de la densité optique à 280 nm).

### EXEMPLE 7:

### Obtention de xylo-oligosaccharides à partir de rafles de maïs broyées.

1 g ( poids sec ) de poudre de rafles de maïs ( granulométrie > 100 mesh) ont été suspendus dans 100 ml de tampon acétate de sodium 50 mM pH 6 contenant 1000 U.I. de xylanase et incubés à 60°C pendant 18 heures.

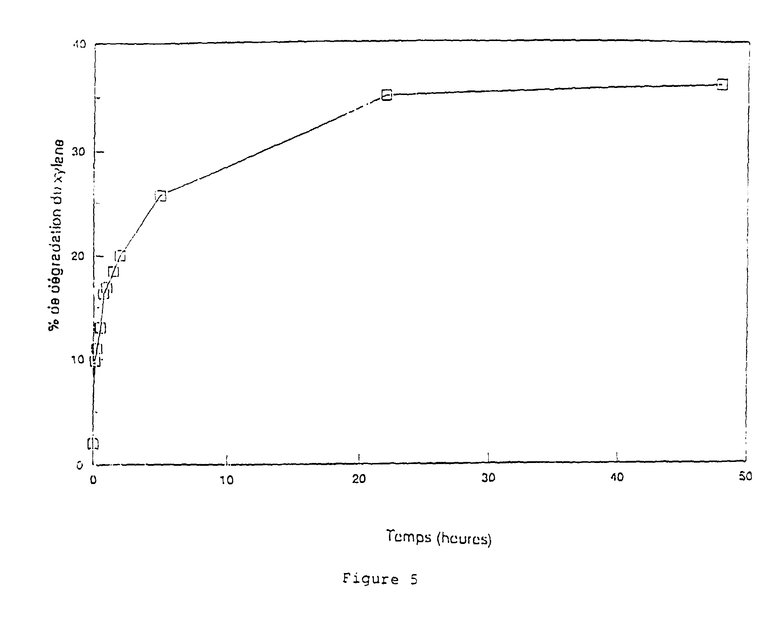

La figure 5 montre qu'il est possible dans ces conditions de transformer jusqu'à 38% du xylane en oligosaccharides (xylo-oligosaccharides et arabinoxylo-oligosaccharides ) . Ces composés peuvent être utilisés tels quels ou comme précurseurs de xylose et d'arabinose.

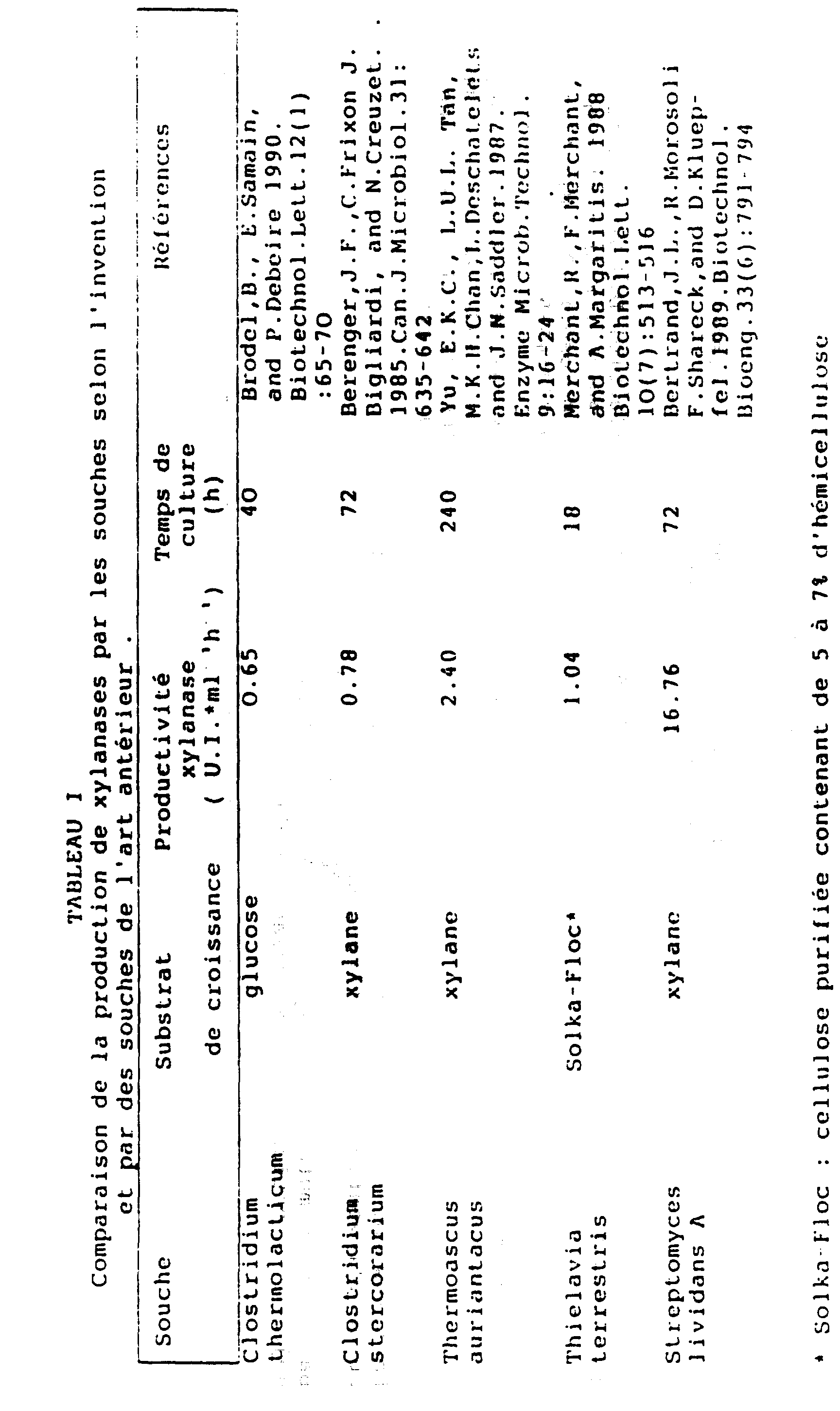

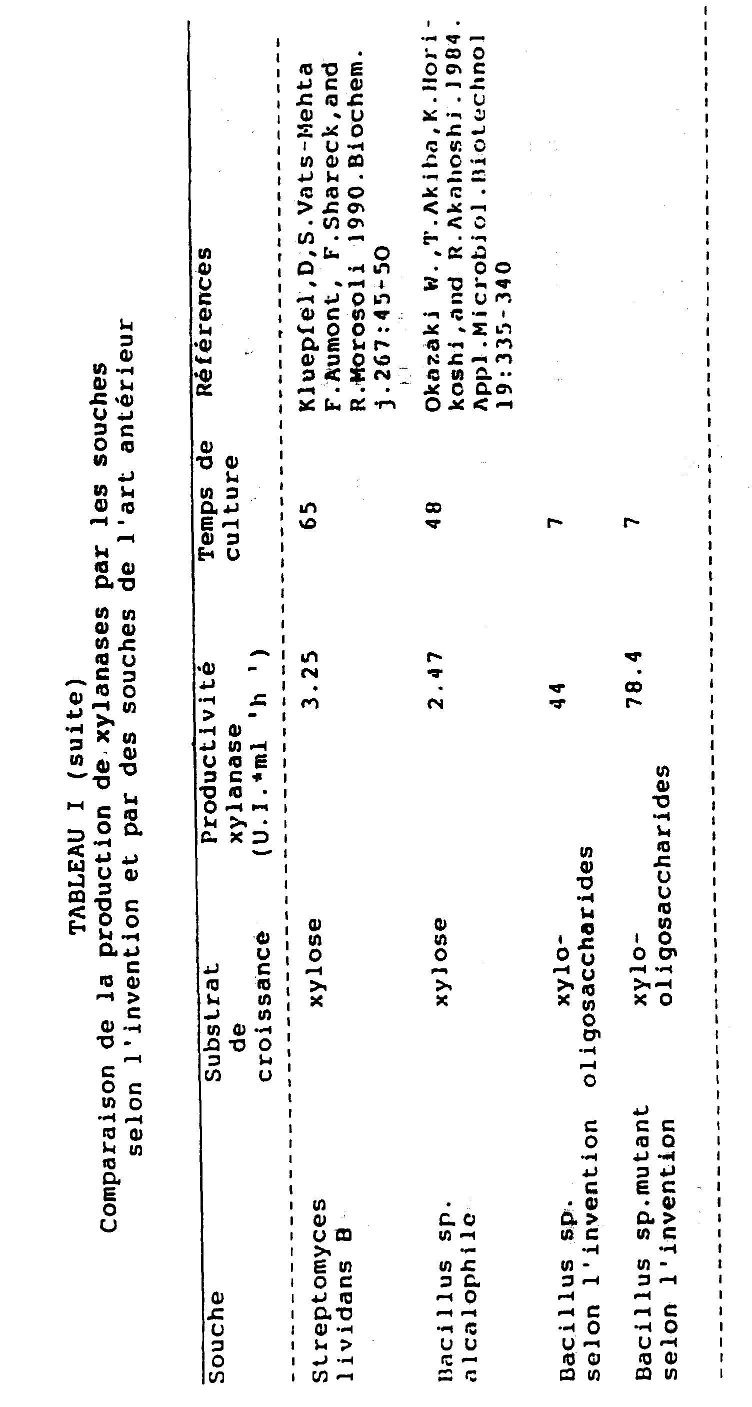

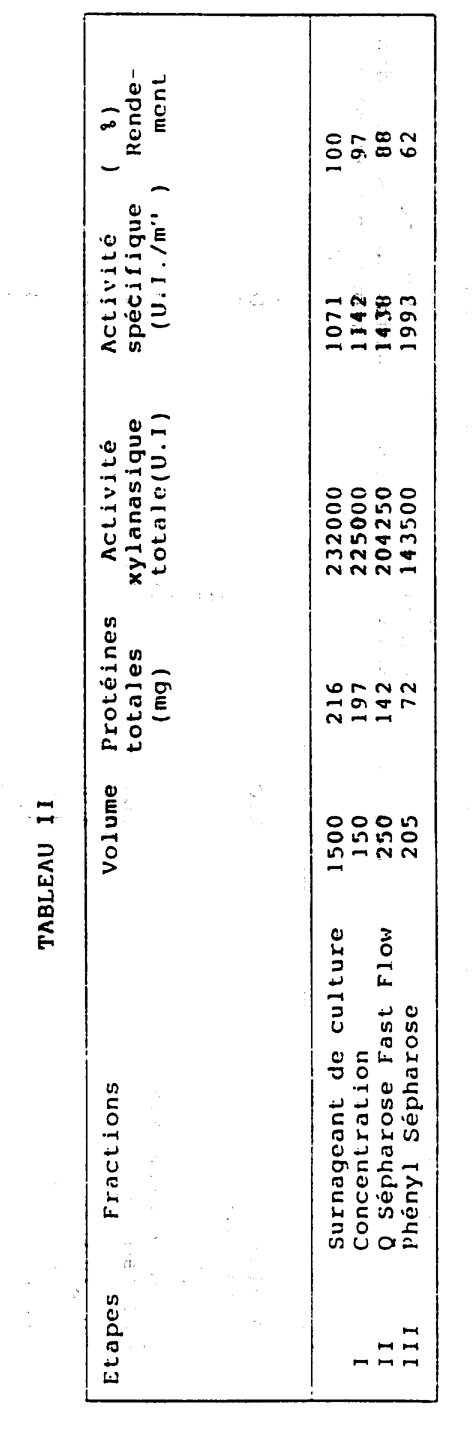

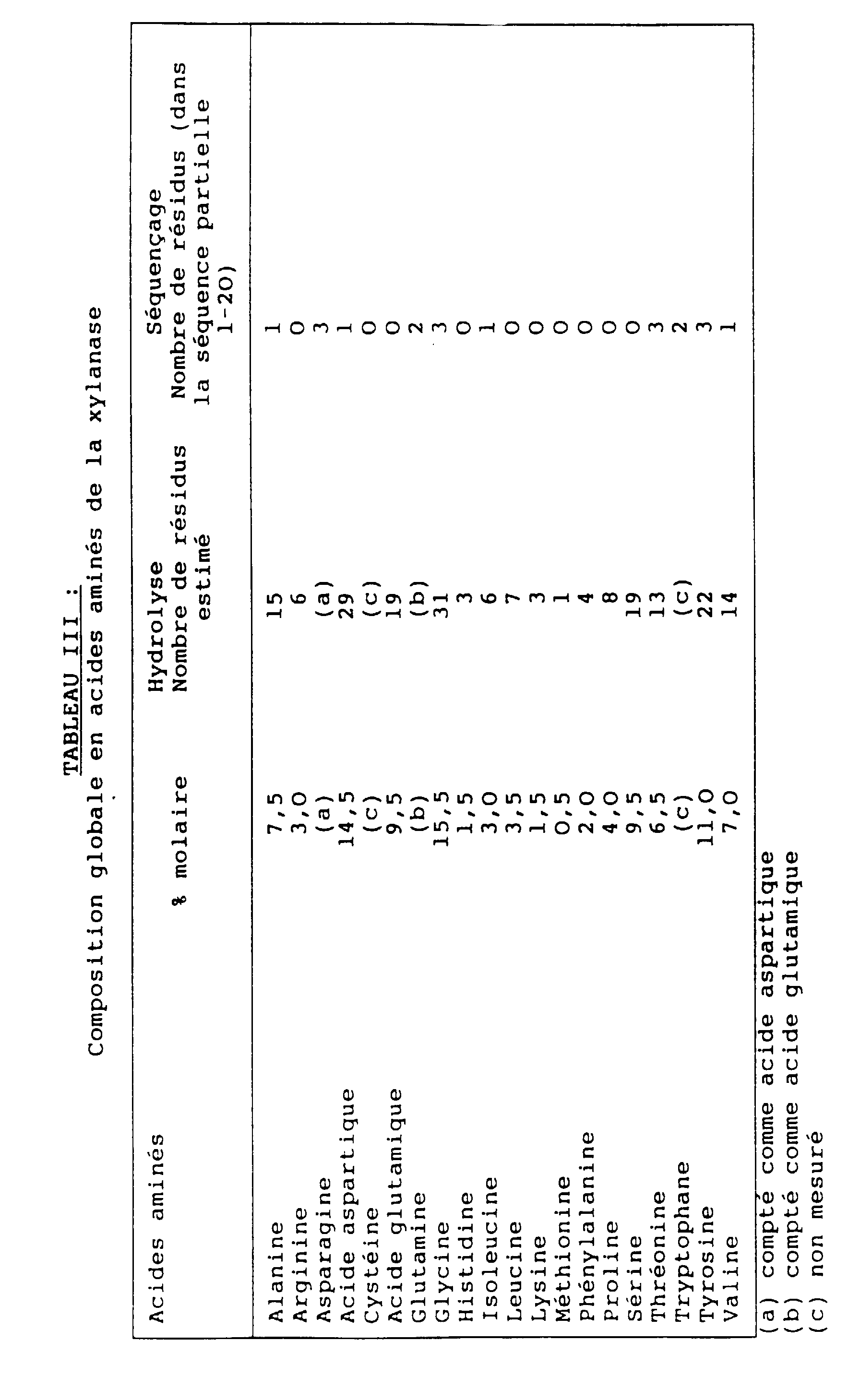

## Revendications

1. Xylanase thermophile, caractérisée en ce qu'elle est stable à 60°C environ, en ce qu'elle possède une masse moléculaire de 22 kDa environ, en ce qu'elle présente un point isoélectrique d'environ 7,7 et en ce qu'elle présente la séquence d'acides aminés N-terminale suivante:

Asn-Thr-Tyr-Trp-Gln-Tyr-Trp-Thr-Asp-Gly-Ile-Gly-Tyr-
Val-Asn-Ala-Thr-Asn-Gly-Gln.

2. Xylanase selon la revendication 1 caractérisée en ce que son pH d'activité optimale est compris dans la gamme allant de 4,8 à 7 et est préférentiellement d'environ 6.

3. Xylanase selon l'une des revendications 1 et 2 caractérisée en ce qu'elle comprend dans sa séquence 15 alanines, 6 arginines, 29 aspartates et asparagines, 19 glutamates et glutamines, 31 glycines, 3 histidines, 6 isoleucines, 7 leucines, 3 lysines, 1 méthionine, 4 phénylalanines, 8 prolines, 19 sérines, 13 thréonines, 22 tyrosines et 14 valines.

4. Souche de Bacillus productrice de xylanase enregistrée sous le n° I-1017 auprès de la Collection Nationale de Cultures des Microorganismes de l'Institut Pasteur.

5. Souche de Bacillus productrice de xylanase enregistrée sous le n° I-1018 auprès de la Collection Nationale de Cultures des Microorganismes de l'Institut Pasteur.

6. Xylanase caractérisée en ce qu'elle est secrétée par l'une des souches de Bacillus selon l'une des revendications 4 et 5.

7. Procédé d'obtention de xylanase selon la revendication 6, comprenant les étapes suivantes :
- concentration de surnageant de culture de Bacillus selon l'une des revendications 4 ou 5,
- passage sur colonne échangeuse d'ions,
- passage sur colonne d'interactions hydrophobes.

8. Procédé de production de xylanase selon la revendication 6 comprenant les étapes de:
- croissance des Bacillus selon l'une des revendications 4 ou 5 dans un milieu contenant un substrat de croissance tel que du glucose, et
- production de xylanase induite par alimentation en continu de la culture par des xylooligosaccharides en quantités appropriées.

9. Utilisation de la xylanase selon l'une des revendications 1 à 3 et 6 dans le blanchiment de la pâte à papier.

10. Utilisation de la xylanase selon l'une des revendications 1 à 3 et 6 dans la préparation de xylooligosaccharides à partir de matières premières d'origine végétale.

## Claims

1. Thermophilic xylanase, characterised in that it is stable at approximately 60°C, it possesses a molecular mass of approximately 22 kDa, it shows an isoelectric point of approximately 7.7 and it possesses the following N-terminal amino acid sequence:Asn-Thr-Tyr-Trp-Gln-Tyr-Trp-Thr-Asp-Gly-Ile-Gly-Tyr-Val-Asn-Ala-Thr-Asn-Gly-Gln.

2. Xylanase according to claim 1 characterised in that its pH of optimal activity is within the range extending from 4.8 to 7, and is preferably approximately 6.

3. Xylanase according to one of claims 1 and 2, characterised in that it comprises in its sequence 15 alanines, 6 arginines, 29 aspartates and asparagines, 19 glutamates and glutamines, 31 glycines, 3 histidines, 6 isoleucines, 7 leucines, 3 lysines, 1 methionine, 4 phenylalanines, 8 prolines, 19 serines, 13 threonines, 22 tyrosines and 14 valines.

4. Bacillus strain productive of xylanase, registered under N°I-1017 with the Collection Nationale de Cultures des Microorganismes de l'Institut Pasteur.

5. Bacillus strain productive of xylanase, registered under n°I-1018 with the Collection Nationale de Cultures des Microorganismes de l'Institut Pasteur.

6. Xylanase characterised in that it is secreted by one of the Bacillus strains according to one of claims 4 and 5.

7. Process for obtaining xylanase according to claim 6 comprising the following steps:
- concentration of the culture supernatant of a Bacillus strain according to one of the claims 4 and 5,
- passage through an ion exchange column;
- passage through a hydrophobic interaction column.

8. Process for the production of xylanase according to claim 6 , comprising the steps:
- of growth of the Bacillus strains according to one of the claims 4 or 5 in a medium containing a growth substrate such as glucose, and
- of production of xylanase induced by feeding the culture continuously with suitable amounts of xylooligosaccharides.

9. Use of the xylanase according to one of claims 1 to 3 and 6 in the bleaching of paper pulp.

10. Use of the xylanase according to one of claims 1 to 3 and 6 in the preparation of xylooligosaccharides from raw materials of plant origin.

## Patentansprüche

1. Thermophile Xylanase, dadurch gekennzeichnet, daß sie bei ungefähr 60°C stabil ist, daß sie eine Molmasse von ungefähr 22 kDa besitzt, daß sie einen isoelektrischen Punkt von ungefähr 7,7 hat und daß sie die folgende N-terminale Aminosäuresequenz aufweist:

Asn-Thr-Tyr-Trp-Gln-Tyr-Trp-Thr-Asp-Gly-Ile-Gly-Tyr-
Val-Asn-Ala-Thr-Asn-Gly-Gln.

2. Xylanase gemäß Anspruch 1, dadurch gekennzeichnet, daß ihr pH-Wert der optimalen Aktivität im Bereich von 4,8 bis 7 liegt und vorzugsweise ungefähr 6 beträgt.

3. Xylanase gemäß einem der Ansprüche 1 und 2, dadurch gekennzeichnet, daß sie in ihrer Sequenz 15 Alaninreste, 6 Argininreste, 29 Aspartat- und Asparaginreste, 19 Glutamat- und Glutaminreste, 31 Glycinreste, 3 Histidinreste, 6 Isoleucinreste, 7 Leucinreste, 3 Lysinreste, 1 Methioninrest, 4 Phenylalaninreste, 8 Prolinreste, 19 Serinreste, 13 Threoninreste, 22 Tyrosinreste und 14 Valinreste umfaßt.

4. Xylanase erzeugender *Bacillus-Stamm,* der bei der Collection Nationale de Cultures des Microorganismes de l'Institut Pasteur unter der Nr. I-1017 hinterlegt ist.

5. Xylanase erzeugender *Bacillus-Stamm,* der bei der Collection Nationale de Cultures des Microorganismes de l'Institut Pasteur unter der Nr. I-1018 hinterlegt ist.

6. Xylanase, dadurch gekennzeichnet, daß sie von einem der *Bacillus-Stämme* gemäß einem der Ansprüche 4 und 5 sezerniert wird.

7. Verfahren zur Gewinnung von Xylanase gemäß Anspruch 6, das die folgenden Schritte umfaßt:
- Konzentrieren des Überstandes einer *Bacillus-Kultur* gemäß einem der Ansprüche 4 oder 5;
- Durchlaufenlassen durch eine Ionenaustauschersäule;
- Durchlaufenlassen durch eine hydrophobe Säule.

8. Verfahren zur Gewinnung von Xylanase gemäß Anspruch 6, das die Schritte umfaßt:
- Züchten eines *Bacillus* gemäß einem der Ansprüche 4 oder 5 in einem Medium, das ein Wachstumssubstrat, wie Glucose, enthält; und
- Produktion von Xylanase, die durch kontinuierliche Zufuhr geeigneter Mengen von Xylooligosacchariden zu der Kultur induziert wird.

9. Verwendung der Xylanase gemäß einem der Ansprüche 1 bis 3 und 6 bei der Bleichung von Papierfaserbrei.

10. Verwendung der Xylanase gemäß einem der Ansprüche 1 bis 3 und 6 bei der Herstellung von Xylooligosacchariden aus pflanzlichen Rohstoffen.
